# EUROPEAN PATENT SPECIFICATION

(11) **EP 2 958 483 B1**
(45) Date of publication and mention of the grant of the patent: **08.04.2020**
(21) Application number: 14754092.6
(22) Date of filing: 20.02.2014
(51) Int. Cl.: A61B 1/24, A61C 19/00, A61B 90/16, A61B 1/06

(54) **ILLUMINATED DENTAL PROP**
BELEUCHTETE ZAHNÄRZTLICHE STÜTZE
ÉCARTEUR DENTAIRE LUMINEUX

(30) Priority: 20.02.2013 US 201313771597
(43) Date of publication of application: 30.12.2015
(73) Proprietor: Khouri, Louie, Birmingham, MI 48009 (US)
(72) Inventor: Khouri, Louie, Birmingham, MI 48009 (US)
(74) Representative: Hoffmann Eitle
(86) International application number: PCT/US2014/017411
(87) International publication number: WO 2014/130680

(56) References cited:
- EP-A2- 2 404 569
- JP-A- 2010 049 902
- KR-A- 20110 085 514
- KR-A- 20120 068 339
- KR-B1- 100 768 030
- US-A- 2 528 458
- US-A1- 2005 239 017
- US-A1- 2008 096 155

## Description

### BACKGROUND

### 1. Field of the Invention

The present invention generally relates to illuminated dental props for holding the mouth open during dental procedures. Dental props, per se, are known to come in a variety of shapes and sizes such as wedge-shaped props and C-shaped props by way of non-limiting example. Generally the props are placed between the upper and lower teeth, opposite the side of the mouth which needs to be accessed by a dental practitioner. However, with regard to accessing and viewing the target area by the dental practitioner, currently available dental props do nothing to enhance the visibility within the oral cavity to carryout the necessary procedure.

Further, while various light sources are available to assist in illuminating the oral cavity, such devices are positioned outside of the oral cavity with the light source directed at the target area. However, external light sources tend to be somewhat ineffective. Thus, the present invention relates to the incorporation of a light assembly with a dental prop of desired size and shape.

### 2. Description of the Prior Art

Relatively recently a handful of patents and patent applications directed to the general concept of combining a light source with a dental prop have surfaced. One such patent is US Patent No. 6,332,776 which issued December 6, 2001 to Martin et al. According to one embodiment disclosed, a unitary body formed to include a first cavity having an inclined reflective surface is disclosed. Light projecting from a light source connected to a light conducting cable is projected upon the reflective surface to emit light within the patient's mouth. Under a second embodiment, a dental prop is constructed including a cavity which hosts the lighting elements including a primary induction coil connected to a secondary induction coil. Under all embodiments disclosed, there does not appear to be any teaching or disclosure of a light assembly which is conveniently detachable from the body of the dental prop.

Alternatively, US Patent Publication No. US/2005/0239018 discloses a lighted dental prop wherein the light source is integrated in a permanently fixed relationship with the body of the bite block. Under this scenario, either the entire construction would be discarded after a single use or the product as a whole is sterilized for reuse. There does not appear to be any disclosure as to replacement of the light source if need be which is another apparent design flaw.

A perceived problem with each of the above-referenced teachings is that the light source is not readily removable from the bite block such that the bite block portion can be sterilized or discarded after a single use. Further, the light assembly is either integral with the bite block portion or requires extreme work to detach the same from the bite block.

### SUMMARY

According to the present invention, there is provided an illuminated dental prop for lighting a patient's mouth comprising: a selectively removable light assembly including a housing, a battery and a light source; and a bite prop which houses said lighting assembly during use, said bite prop having a body including a teeth engaging surface for maintaining the patient's mouth in an open position and an inner wall defining an opening for receiving the light assembly, said dental prop and said removable light assembly being of a size and geometry to be fully contained within the patient's mouth to thereby maintain the patient's mouth in an open position during use, wherein the bite prop includes first and second legs extending from a transverse portion which defines a substantially C-shaped opening for receiving the light assembly, characterized in that: the removable light assembly comprises at least one flange which has offset sides; and at least one of said first and second legs includes a truncated wall extending into the opening to hold the light assembly and adapted to seat against the offset sides when the light assembly is fully inserted into the opening.

The present invention provides for an illuminated dental prop for holding the dental patient's mouth open during dental procedures which incorporates a selectively removable light source. In addition to providing much needed light to the oral cavity of a patient, a significant advantage over the above noted references is the ability to readily remove the light assembly from the bite block to facilitate sterilization of the light assembly and, under certain embodiments, discarding of the bite block portion. The dental props of the present invention are designed to be of a size and geometry to be fully contained within the patient's mouth, i.e., without wires extending out of the mouth, to ensure clearance in the oral cavity of the patient such that the practitioner can access the target area with the necessary dental instruments.

### DRAWINGS

The drawings described herein are for illustration purposes only and are not intended to limit the scope of the present disclosure in any way.
Fig. 1 is a perspective view of a first example of an illuminated dental prop assembly not forming part of the invention as claimed;
Fig. 2 is a blown apart perspective view of the illuminated dental prop of Fig. 1;
Fig. 3 is a sectional view of the illuminated dental prop assembly of Figs. 1 and 2;
Fig. 4 is a sectional view of an alternative illuminated dental prop not forming part of the invention as claimed and featuring a different lighting assembly;
Fig. 5 is a perspective view of an alternate illuminated dental prop not forming part of the invention as claimed;
Fig. 6 is a perspective view of an alternative illuminated dental prop not forming part of the invention as claimed wherein the light assembly is retro-fit to an existing conventional dental prop;
Fig. 7 is an alternative illuminated dental prop assembly not forming part of the invention as claimed including a lighted sleeve disposable over the body of a dental prop;
Fig. 8 is an alternative illuminated dental prop assembly not forming part of the invention as claimed including a lighted pliable attachment disposable over the body of a dental prop;
Fig. 9 is an alternative illuminated dental prop assembly not forming part of the invention as claimed including a lighted band disposable over the body of a dental prop;
Fig. 10 is an embodiment of an illuminated dental prop assembly according to the invention including a dental prop and a selectively removable light assembly;
Fig. 11 is a perspective view of the embodiment of Fig. 10;
Fig. 12 is a perspective view showing the dental prop and light assembly separated;
Fig. 13 shows dental props of different sizes which are capable of hosting a standard light assembly; and
Fig. 14 shows an embodiment when the light assembly is rechargeable.

### DETAILED DESCRIPTION

The following description is merely exemplary in nature and is not intended to limit the present disclosure, application, or uses. It should be understood that throughout the drawings, corresponding reference numerals indicate like or corresponding parts and features.

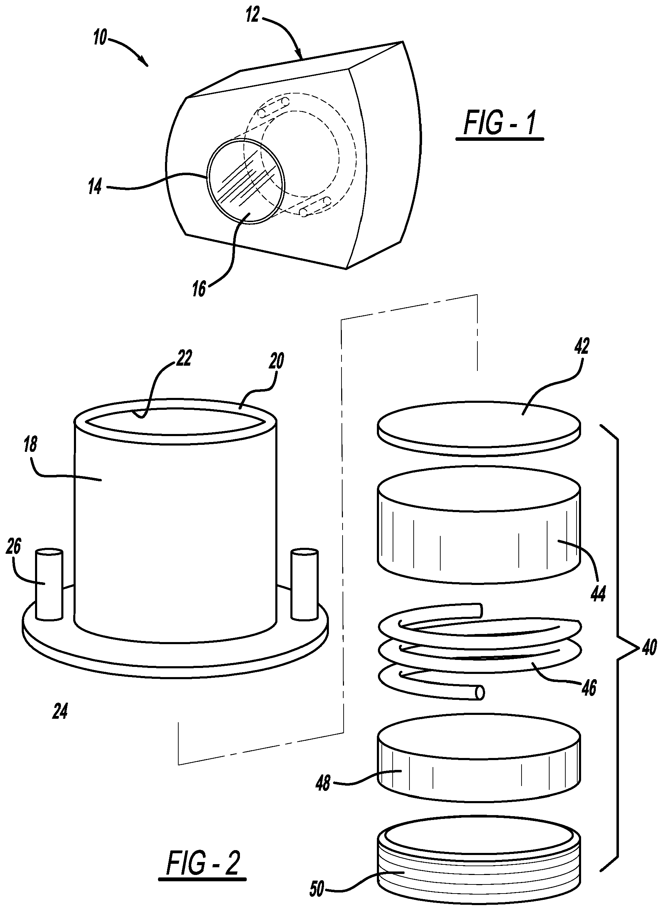

A first example of an illuminated dental prop not forming part of the present invention is shown with reference to Figs. 1 -3. Initially, referring to Fig. 1 , there is shown the illuminated dental prop 10 which includes a body 12 which forms the bite block portion of the assembly and a light assembly 16 which is mounted through an aperture 14 of the body 12. The body 12 can be in many shapes or configurations and is shown according to the first example as a wedge-shaped bite block having upper and lower teeth engaging surfaces. The light assembly 16 which is selectively detachable from the body of the bite block generally includes a housing 18 and a light source 40. The housing 18 includes a sleeve 20 including a longitudinally central bore 22 for hosting the light source 40. The sleeve 20 includes in inwardly extending lip 28 at one end and a threaded interior lead-in portion 30 along the opposite end of lip 28. The housing 18 also includes an integral outwardly extending flange disposed along the end opposite lip 28 which may optionally include one or more posts 26 which seat into the body 12 of the bite block to assist in securing the lighting assembly.

The light source 40 according to the first example 10 generally includes a lens 42 disposed against the inwardly extending lip 28 of the housing 18, a lighting element 44, an electrical contact 46, a battery 48, and a threaded end cap 50. As should be understood by those skilled in the art, as the threaded end cap 50 is rotated to an extent such that the battery is in sufficient electrical contact with the lighting element through electrical contact 46, the lighting element 44 becomes illuminated and remains illuminated until such time that the threaded end cap is sufficiently backed away. According to this example, the lighting element 44 is in the form of a miniature light bulb; however, as will be described in further detail below, alternative lighting means may be utilized.

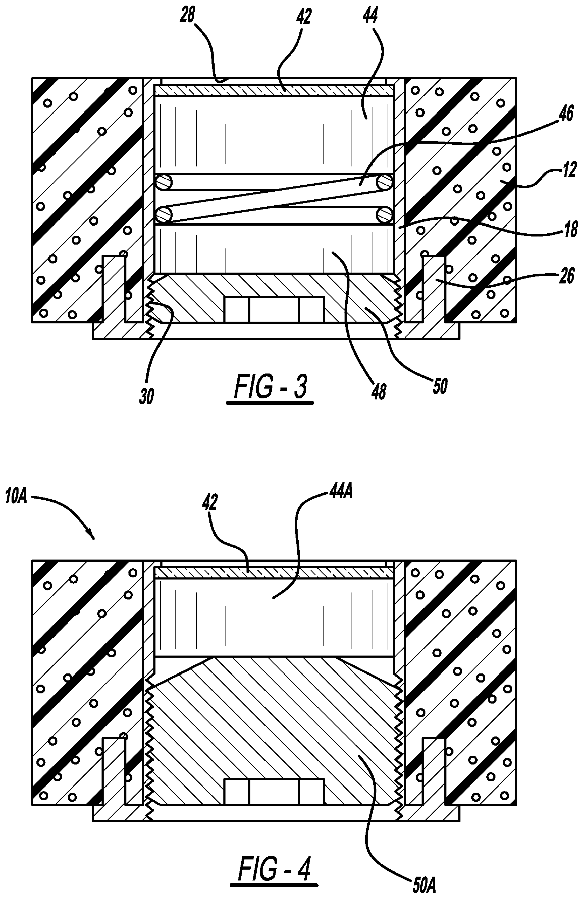

Referring to Fig. 4, there is shown a first alternative illuminated dental prop example 10A not forming part of the claimed invention, wherein the lighting element 44A is activated by the threaded end cap 50A which is in the form of an induction element. As should be understood by those skilled in the art, the induction element 50A is activated by another induction coil (not shown) via electromagnetic energy.

In addition to the dental props shown in Figs. 1 -3 and 4, respectively, it should be understood that the lighting assembly may be employed with those or other dental prop bodies. For example, the lighting assembly may be an LED light package including an LED light source and a battery. This assembly may be two separate components or may be in the form of a self-contained assembly which is attached to the dental prop body. Whatever light assembly is employed, it is envisioned the light assembly will have a useful life which affords the end user the option of reusing the light assembly after appropriate sterilization by inserting it into a fresh dental prop body. Thus, a kit having at least one selectably reusable light assembly along with a plurality of disposable dental props may be provided commercially.

Alternately, a reusable dental prop with a plurality of detachable, disposable light assemblies is also envisioned.

Regardless of the dental prop body design, generally the upper and lower teeth will sit in a stable position on the respective upper and lower teeth engaging surface.

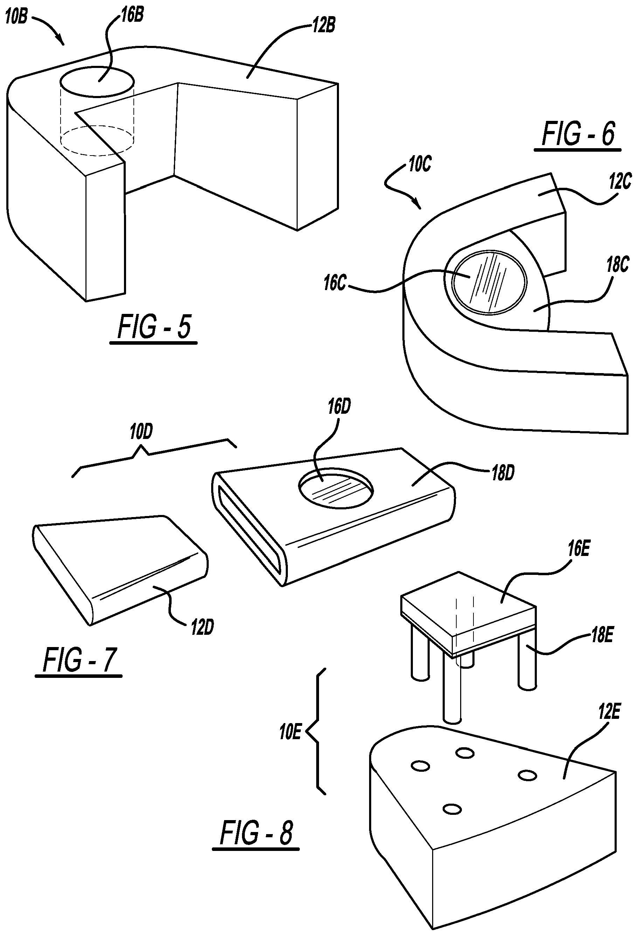

Referring to Fig. 5, there is yet another alternative example 10B not forming part of the invention as claimed which includes a lighting assembly 16B disposed within an opening along a central portion of the body 12B of the bite block assembly. As can be seen from Fig. 5, the body is substantially C-shaped such that the bite block provides sufficient clearance so that it does not interfere with the dental practioner during a dental procedure. Thus, the C-shaped body is defined by first and second forward projecting portions which are engaged by the teeth and a central body portion which is positioned toward the back of the mouth.

Referring to Fig. 6, yet another alternative example 10C not forming part of the invention as claimed is depicted. The body 12C also generally has a C-shaped body and the lighting assembly 16C, rather than being embedded within the body, is in the form of a selectively detachable unit which is mounted within the concave recess along the interior wall of the body 12C. This embodiment is considered to be an option for retro-fitting currently commercially available bite blocks. The lighting assembly 16C may be mechanically attached via a male and female coupling with the body or adhesively attached within the recess.

Referring to the example of Fig. 7 referenced by the numeral 10D and not forming part of the invention as claimed, the bite block body 12D fits within a pliable sheath. The sheath serves as the housing 18D and hosts the lighting assembly 16D.

Referring to Fig. 8, another example 10E not forming part of the invention as claimed is depicted wherein the body 12E includes a plurality of openings for receiving the platform-type housing 18E having mounted thereto a light assembly 16E.

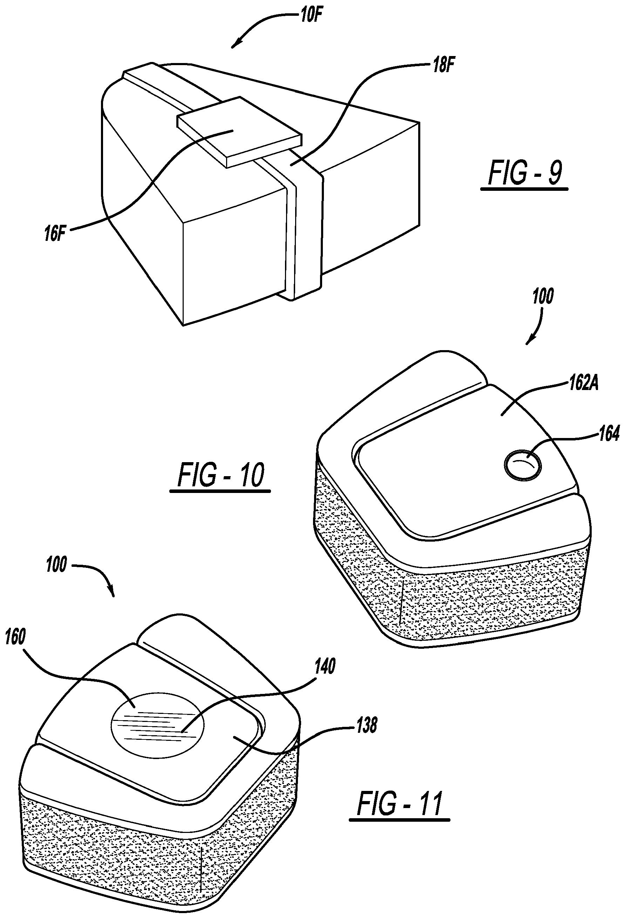

Referring to Fig. 9, another example 10F not forming part of the invention as claimed is shown as including a housing 18F in the form of a band having attached thereto a light assembly 16F. The band can be formed from various materials including elastic by way of non-limiting example. This type of structure would be ideal for retro-fitting commercially available bite blocks including wedges as well as C-shaped bite blocks with lighting to illuminate the oral cavity.

The illuminated dental props of Figs. 6-9 preferably employ a self-contained LED light as the light source. As should be understood by those skilled in the art, the LED lighting should illuminate the oral cavity to an appreciable extent but, should not generate light that falls within the light range that would promote undesired curing of any materials employed in the dental procedure being carried out. Additionally, the lighting assembly shall not generate as unacceptable level of heat which could be uncomfortable to a patient.

The body 12C which is generally formed from a moldable thermoplastic, thermoset or elastomeric material may include a slot (not shown) along the concave recess into which the lighting assembly 16C is press fit. The slot may include a locking mechanism such that when the lighting assembly is disposed therein the battery will be activated.

Referring to Figs 10-12, there is shown anilluminated dental prop embodiment 100 according to the present invention and including two major components, a bite prop 112 and a light assembly 114. The light assembly, as with other examples shown herein, includes as its main components a housing 138 and a lighting source 140. The housing can be formed from a variety of materials sufficient to protect the lighting package secured within, such as lightweight thermoplastics, thermosets or silicone, by way of non-limiting example. The light source is preferably a LED light.

As shown, the lighting assembly housing 138 has an exterior wall 144 defined by a front wall 146, rear wall 148 and opposing top and bottom walls 150, 150A, respectively. The top and bottom walls in the embodiment shown are mirror images, both including a recess 152 which receives the tabs 136 projecting from the interior wall of the bite prop. The top and bottom walls also include a flange 154 located proximate to the front wall 146 which has offset sides 156. As also shown, the light assembly includes a translucent area 160 through which the light generated by the light assembly is transmitted. The area can be limited to help focus the light or can take up a larger portion of the sidewall 162. The light assembly will also typically include an on/off button or switch 164 shown here on the opposing sidewall 162A.

The bite prop 112 has a substantially C-shaped body including first and second legs, 116 and 116A, respectively, extending at an inclined angle from the traverse portion 118 to define an open area 132 which ultimately is occupied by the light assembly. This slight angle allows the light assembly to be more easily inserted into the opening or open area for engagement with the bite prop. The external wall 120 extending along the transverse portion 118 and first and second legs 116, 116A of the prop 100, optionally, but preferably, includes an enhanced tooth engaging surface 122. Thus, when the dental prop 100 is positioned over the patient's teeth at the back of the mouth, with the transverse portion near the back and the first and second legs projecting toward the front of the mouth, the extra grip provided along the external wall 120, in association with the patient's natural instinct to close their jaw, helps keep the prop fixed over the teeth. The tooth engaging surface 122 can be formed as an integral part of the body in the form of a roughened surface or may be an over-molded piece of elastomeric material having a roughened surface.

The interior wall 124 of the bite prop which extends along the transverse portion 118 and the first and second legs 116, 116A is sized to tightly accommodate the light assembly 114 when it is fully inserted therein. As such, the interior wall 124 is in close contact with the exterior wall 144 of the light assembly 114 during use. An enhanced locking feature is also offered by one or more tabs 136 extending from the interior wall which mate with a complementary recess 152 provided along the exterior wall of the light assembly. As shown, it is preferable that there be at least one tab along the first and second legs and at least one recess along the top and bottom walls of the exterior wall. While not shown, it should be understood that the interior wall may have at least one recess and the exterior wall of the light assembly may host one or more locking tabs.

Yet another feature of the bite prop are the truncated sidewalls 130 that extend from at least one and preferably both of the legs 116 and 116A into the opening 132 which is ultimately filled by the light assembly during use.

The truncated sidewalls 130 seat against the offset sides 156 of the flange 154 of the light assembly body when the light assembly is fully inserted into the opening 132. As should be appreciated, while the overall design of the illuminated dental prop makes it easy to mate and remove the light assembly from the dental prop, it is highly secure when the light assembly is fully engaged within the opening of the dental prop.

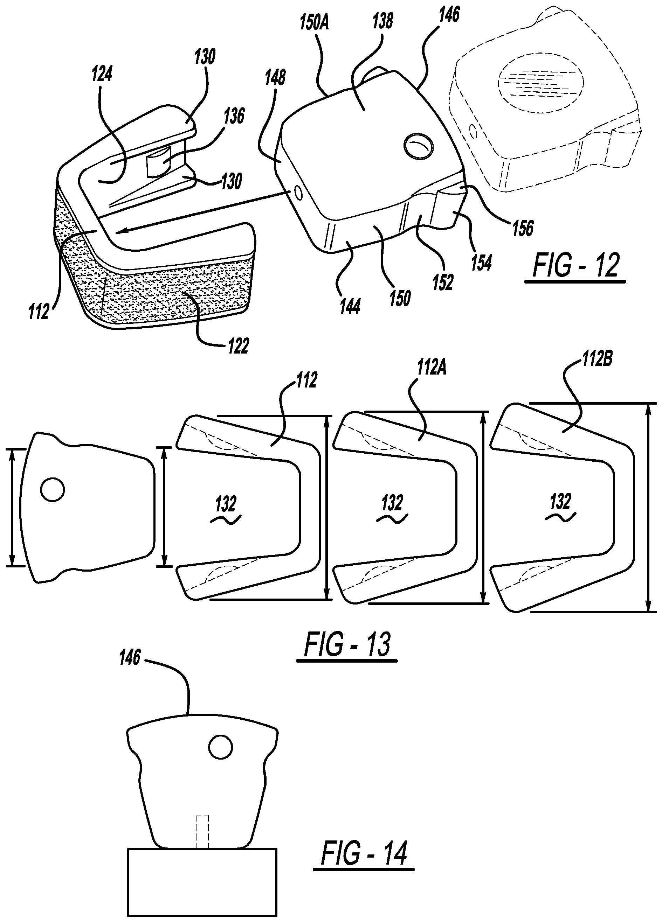

Referring to Fig. 13, there are shown a plurality of bite props 112, 112A and 112B each of which are progressively larger and serve to accommodate patient's having small, medium and large sized oral cavities. By design, the inner dimension, e.g., the inner wall of the bite prop, remains the same size so that a single light assembly can be used with dental props sized to different patients. Thus, the illuminated dental prop can be offered as a kit.

Referring to Fig. 14, it is demonstrated that under one embodiment, the light assembly includes a receptacle for receiving a battery charger, however, it is also contemplated that the light assembly can be of the type which is capable of being recharged using an induction type system. Still further, as discussed above, the light assembly may include replaceable batteries or may be replaced with another light assembly when the charge of the batteries is exhausted.

The description of the invention is merely exemplary in nature and, thus, variations that do not depart from the scope of the claims are intended to form part of the invention.

## Claims

1. An illuminated dental prop (100) for lighting a patient's mouth comprising:
a removable light assembly (114) including a housing (138) and a light source (140); and
a bite prop (112) which houses said lighting assembly (138) during use, said bite prop having a body including a teeth engaging surface (122) for maintaining the patient's mouth in an open position and an inner wall (124) defining an opening for receiving the light assembly, said dental prop and said removable light assembly (138) being of a size and geometry to be fully contained within the patient's mouth to thereby maintain the patient's mouth in an open position during use, wherein the bite prop (112) includes first and second legs (116, 116A) extending from a transverse portion (118) which defines a substantially C-shaped opening (132) for receiving the light assembly (138),
**characterized in that**:
the removable light assembly (138) comprises at least one flange (154) which has offset sides (156); and
at least one of said first and second legs (116, 116A) includes a truncated wall (130) extending into the opening (132) to hold the light assembly (138) and adapted to seat against the offset sides (156) when the light assembly (138) is fully inserted into the opening (132).

2. The illuminated dental prop of claim 1 wherein said light source is an LED light.

3. The illuminated dental prop of claim 1 wherein the housing (138) of the light assembly (114) is formed from a material selected from the group consisting of thermoplastic, thermosets and silicone.

4. The illuminated dental prop of claim 1 wherein the housing (138) of the light assembly (114) includes a translucent portion through which light generated by the light source (140) is transmitted.

5. The illuminated dental prop of claim 1 wherein said bite prop (112) includes the inner wall (124) occurring along the first and second legs (116, 116A) and said transverse portion (118), said inner wall including at least one inwardly projecting tab (136) which engages the light assembly housing (138) to further secure the light assembly (114) in contact with said bite prop (112).

6. The illuminated dental prop of Claim 1, wherein the light assembly housing (138) has a translucent portion.

7. The illuminated dental prop of claim 6 wherein the truncated wall (130) includes a pair of spaced sidewalls occurring along at least one of the first and second legs (116, 116A).

8. The illuminated dental prop of claim 7 wherein both the first and second legs (116, 116A) include at least one truncated wall (130).

9. The illuminated dental prop of claim 6 wherein said inner wall (124) includes a tab (136) extending toward the open area (132) and the light assembly housing (138) includes a recess (152) which mates with the tab (136) of the bite prop upon sufficient insertion of the light assembly (114) into the open area (132) of the bite prop (112).

10. The illuminated dental prop of claim 9 wherein the tab (136) occurs along the inner wall (124) along one of said first and second legs (116, 116A).

11. The illuminated dental prop of claim 10 wherein said bite prop (112) includes a tab (136) on both said first and second legs (116, 116A) which mate with corresponding recesses (152) on the light assembly housing (138).

## Patentansprüche

1. Beleuchtete Dentalstütze (100) zum Beleuchten des Mundes eines Patienten, umfassend:
eine abnehmbare Lichtmontage (114), die ein Gehäuse (138) und eine Lichtquelle (140) einschließt; und
eine Beißstütze (112), die die Beleuchtungsanordnung (138) im Gebrauch aufnimmt, wobei die Beißstütze einen Körper aufweist, der eine Zahnauflagefläche (122) zum Halten des Mundes des Patienten in einer offenen Stellung und eine Innenwand (124), die eine Öffnung zum Aufnehmen der Lichtmontage definiert, einschließt, wobei die Dentalstütze und die abnehmbare Lichtanordnung (138) eine derartige Größe und Geometrie aufweisen, dass sie vollständig in dem Mund des Patienten enthalten sind, um so im Gebrauch den Mund des Patienten in einer offenen Stellung zu halten, wobei die Beißstütze (112) ein erstes und ein zweites Bein (116, 116A) einschließt, die sich von einem querlaufenden Abschnitt (118), der eine im Wesentlichen C-förmige Öffnung (132) zum Aufnehmen der Lichtmontage (138) definiert, erstrecken,
**dadurch gekennzeichnet, dass**:
die abnehmbare Lichtmontage (138) zumindest einen Flansch (154) umfasst, der gekröpfte Seiten (156) aufweist; und
zumindest eines des ersten und des zweiten Beins (116, 116A) eine abgestumpfte Wand (130) einschließt, die sich in die Öffnung (132) erstreckt, um die Lichtmontage (138) zu halten, und dazu eingerichtet ist, an den gekröpften Seiten (156) aufzuliegen, wenn die Lichtmontage (138) vollständig in die Öffnung (132) eingesetzt ist.

2. Beleuchtete Dentalstütze nach Anspruch 1, wobei die Lichtquelle ein LED-Licht ist.

3. Beleuchtete Dentalstütze nach Anspruch 1, wobei das Gehäuse (138) der Lichtmontage (114) aus einem Material gebildet ist, das aus der Gruppe ausgewählt ist, die aus Thermoplast, Duroplasten und Silikon besteht.

4. Beleuchtete Dentalstütze nach Anspruch 1, wobei das Gehäuse (138) der Lichtmontage (114) einen durchscheinenden Abschnitt einschließt, durch den von der Lichtquelle (140) erzeugtes Licht übertragen wird.

5. Beleuchtete Dentalstütze nach Anspruch 1, wobei die Beißstütze (112) die entlang des ersten und des zweiten Beins (116, 116A) auftretende Innenwand (124) und den querlaufenden Abschnitt (118) einschließt, wobei die Innenwand zumindest eine nach innen ragende Leiste (136) einschließt, die das Lichtmontagegehäuse (138) in Eingriff nimmt, um die Lichtanordnung (114) im Kontakt mit der Beißstütze (112) weiter zu sichern.

6. Beleuchtete Dentalstütze nach Anspruch 1, wobei das Lichtmontagegehäuse (138) einen durchscheinenden Abschnitt aufweist.

7. Beleuchtete Dentalstütze nach Anspruch 6, wobei die abgestumpfte Wand (130) ein Paar beabstandeter Seitenwände einschließt, die entlang zumindest eines des ersten und des zweiten Beins (116, 116A) auftreten.

8. Beleuchtete Dentalstütze nach Anspruch 7, wobei sowohl das erste als auch das zweite Bein (116, 116A) zumindest eine abgestumpfte Wand (130) einschließen.

9. Beleuchtete Dentalstütze nach Anspruch 6, wobei die Innenwand (124) eine Leiste (136) einschließt, die sich zu dem offenen Bereich (132) hin erstreckt, und das Lichtmontagegehäuse (138) eine Aussparung (152) einschließt, die bei ausreichendem Einsetzen der Lichtmontage (114) in den offenen Bereich (132) der Beißstütze (112) mit der Leiste (136) der Beißstütze zusammenpasst.

10. Beleuchtete Dentalstütze nach Anspruch 9, wobei die Leiste (136) entlang der Innenwand (124) entlang eines des ersten und des zweiten Beins (116, 116A) auftritt.

11. Beleuchtete Dentalstütze nach Anspruch 10, wobei die Beißstütze (112) eine Leiste (136) auf sowohl dem ersten als auch dem zweiten Bein (116, 116A) einschließt, die mit entsprechenden Aussparungen (152) auf dem Lichtmontagegehäuse (138) zusammenpassen.

## Revendications

1. Écarteur dentaire lumineux (100) pour éclairer la bouche d'un patient comprenant :
un ensemble d'éclairage amovible (114) comprenant un boîtier (138) et une source de lumière (140) ; et
un écarteur occlusal (112) qui abrite ledit ensemble d'éclairage (138) lors de l'utilisation, ledit écarteur occlusal ayant un corps comprenant une surface de mise en prise avec les dents (122) pour maintenir la bouche du patient en position ouverte et une paroi interne (124) définissant une ouverture pour recevoir l'ensemble d'éclairage, ledit écarteur dentaire et ledit ensemble d'éclairage amovible (138) ayant une taille et une géométrie leur permettant de pénétrer complètement dans la bouche du patient pour maintenir ainsi la bouche du patient en position ouverte lors de l'utilisation, dans lequel l'écarteur occlusal (112) comprend des première et seconde pattes (116, 116A) s'étendant à partir d'une partie transversale (118) qui définit une ouverture sensiblement en forme de C (132) pour recevoir l'ensemble d'éclairage (138),
**caractérisé en ce que** :
l'ensemble d'éclairage amovible (138) comprend au moins une bride (154) qui a des côtés décalés (156) ; et
au moins une desdites première et seconde pattes (116, 116A) comprend une paroi tronquée (130) s'étendant dans l'ouverture (132) pour maintenir l'ensemble d'éclairage (138) et conçue pour reposer contre les côtés décalés (156) lorsque l'ensemble d'éclairage (138) est complètement inséré dans l'ouverture (132).

2. Écarteur dentaire lumineux selon la revendication 1 dans lequel ladite source de lumière est une lumière LED.

3. Écarteur dentaire lumineux selon la revendication 1 dans lequel le boîtier (138) de l'ensemble d'éclairage (114) est formé à partir d'un matériau choisi dans le groupe constitué d'un matériau thermoplastique, d'un matériau thermodurcissable et de la silicone.

4. Écarteur dentaire lumineux selon la revendication 1 dans lequel le boîtier (138) de l'ensemble d'éclairage (114) comprend une partie translucide à travers laquelle la lumière générée par la source de lumière (140) est transmise.

5. Écarteur dentaire lumineux selon la revendication 1 dans lequel ledit écarteur occlusal (112) comprend la paroi interne (124) se trouvant le long des première et seconde pattes (116, 116A) et de ladite partie transversale (118), ladite paroi interne comprenant au moins un ergot (136) faisant saillie vers l'intérieur qui vient en prise avec le boîtier de l'ensemble d'éclairage (138) pour renforcer encore le contact de l'ensemble d'éclairage (114) avec ledit écarteur occlusal (112).

6. Écarteur dentaire lumineux selon la revendication 1, dans lequel le boîtier de l'ensemble d'éclairage (138) a une partie translucide.

7. Écarteur dentaire lumineux selon la revendication 6 dans lequel la paroi tronquée (130) comprend une paire de parois latérales espacées se trouvant le long au moins d'une des première et seconde pattes (116, 116A).

8. Écarteur dentaire lumineux selon la revendication 7 dans lequel les première et seconde pattes (116, 116A) comprennent toutes deux au moins une paroi tronquée (130).

9. Écarteur dentaire lumineux selon la revendication 6 dans lequel ladite paroi interne (124) comprend un ergot (136) s'étendant vers la zone ouverte (132) et le boîtier de l'ensemble d'éclairage (138) comprend un évidement (152) qui s'accouple avec l'ergot (136) de l'écarteur occlusal lorsque l'ensemble d'éclairage (114) est suffisamment inséré dans la zone ouverte (132) de l'écarteur occlusal (112).

10. Écarteur dentaire lumineux selon la revendication 9 dans lequel l'ergot (136) se trouve le long de la paroi interne (124) le long de l'une desdites première et seconde pattes (116, 116A).

11. Écarteur dentaire lumineux selon la revendication 10 dans lequel ledit écarteur occlusal (112) comprend un ergot (136) à la fois sur lesdites première et seconde pattes (116, 116A) qui s'accouple avec des évidements (152) correspondants sur le boîtier de l'ensemble d'éclairage (138).
